# EUROPEAN PATENT SPECIFICATION

(11) **EP 4 438 900 B1**
(45) Date of publication and mention of the grant of the patent: **18.03.2026**
(21) Application number: 23166023.4
(22) Date of filing: 31.03.2023
(51) Int. Cl.: F04C 2/22, F04C 5/00, F04C 15/00, A61M 1/00, A61M 5/00

(54) **TROCHOIDAL MEDICAMENT PUMP DEVICE: MATERIAL AND GEOMETRY**
TROCHOIDENFÖRMIGE MEDIKAMENTENPUMPVORRICHTUNG: MATERIAL UND GEOMETRIE
DISPOSITIF TROCHOÏDAL DE POMPE À MÉDICAMENT : MATÉRIAU ET GÉOMÉTRIE

(43) Date of publication of application: 02.10.2024
(73) Proprietor: Medico Invest AG, 9001 St. Gallen (CH)
(72) Inventor: BECHTOLD, Herbert, 78588 Denkingen (DE); GAUL, Stefan, 8234 Stetten (CH); OAKLEY, Tom, Cambridgeshire, CB23 5BH (GB); WYATT, Thomas, Cottenham, CB24 8RG (GB); DE SILVA, Kamaal, Cambridge, CB4 1LQ (GB)
(74) Representative: Maiwald GmbH

(56) References cited:
- WO-A1-2021/109759
- WO-A2-01/39816
- WO-A2-2019/057570
- US-A1- 2018 010 612
- US-A1- 2019 143 039
- US-A1- 2021 353 854
- US-B1- 9 511 186

## Description

### Field of the Invention

The present invention relates to a trochoidal medicament pump device, which has a particular geometry and material selection, and further relates to a giving set having included said trochoidal medicament pump device. The trochoidal medicament pump device and the giving set having included said trochoidal medicament pump device according to the invention provide a trochoidal medicament pump device and giving set having included said trochoidal medicament pump device with improved delivery properties, accuracy, reliability, and lifetime.

### Background of the Invention

Trochoidal pump devices are known for pumping applications, e.g., from US 4,137,024 which describes a rotary piston mechanism such as compressors and expansion engines, and from JP-S-62-17322, which describes a rotary piston type compressor. Trochoidal pump devices are also known for pumping applications in refrigerators, as described e.g., in US 6,520,754, which describes a compressor for a refrigerator for household applications. US 6,168,405 B1 describes a vacuum cleaner with a Wankel-type pump for pumping air wherein the chamber has an epitrochoidal planform satisfying a particular equation.

A trochoidal pump device for medical applications is also known, e.g., from WO 2019/057570, which describes a rotary pump for use in a medicament delivery device. Further, WO 2021/109759 A1 describes a triangular rotor pump, which however does not have any relation to medicament delivery. Further, WO 2019/057570 A2 describes rotary pump and a medical device for the administration of one or more liquid medicaments to a patient, which however does not have any modularity and exchangeability of the pump and its drive. US 9511 186 B1 describes an oval rotor medicament injection system with a pump, which however does not have a trochoidal shape. US 2021/353854 A1 describes an infusion pump with a pump engine, which however does not have a receptacle for a trochoidal pump.

US 2018/010612 A1 describes a flexible impeller pump, which however does not have a trochoidal pump. US 2019/143039 A1 describes an infusion device with different spiral or bent needle shapes. WO 01/39816 A2 describes an infusion pump, which however does not have a trochoidal pump.

Over the known prior art is can be considered as an object to provide a trochoidal medicament delivery device with improved delivery properties, higher accuracy, increased reliability, and longer lifetime.

### Summary of the Invention

The present invention provides a trochoidal medicament pump device and a giving set including said trochoidal medicament delivery device, according to the independent claim, whereas further embodiments are incorporated in the dependent claims.

According to the invention as defined in independent claim 1 there is provided a trochoidal medicament pump device comprising a pump body with an in-port for entering a medicament to be administered and an out-port for administering a medicament, a pump rotor having a triangular convex shape and having an eccentric trajectory upon rotation, i.e. revolving around an axis, within the pump body, wherein the pump rotor has three equidistant apexes, each two thereof are connected with a convex curved line, wherein the pump body has a pump chamber, wherein the pump chamber has a first lobe forming a first inner wall portion of a pump volume of the pump chamber with a first inlet in fluid communication with the in-port and a first outlet in fluid communication with the out-port with the pump rotor eccentrically rotating, i.e. revolving around an axis, so that the rotor with the first wall portion forms upon rotation a first suction volume in fluid communication to the first inlet and a first expelling volume in fluid communication to the first outlet, wherein the pump rotor has a rigid rotor core and an elastic or elastomeric over-mold over at least sealing lines along the apexes and sealing lines along the convex curved lines, wherein the over-mold is made of an elastic or elastomeric material of a first material class, wherein the first inner wall portion is made of a material of a second material class, wherein the outer dimensions of the pump rotor with the over-mold, including a top and bottom, are equivalent or larger than the corresponding inner dimensions of the pump chamber, wherein the apexes of the eccentrically rotating pump rotor trace a trochoidal line corresponding to a trochoidal shape that is equivalent or larger than the corresponding inner dimensions of the pump chamber, wherein the pump chamber has a trochoidal shape with said first lobe and a second lobe forming a second inner wall portion of a pump volume of the pump chamber with the pump rotor eccentrically rotating with the apexes traveling along a trochoidal line corresponding to the trochoidal shape, thus forming upon rotation a second suction volume and a second expelling volume, wherein the second inner wall portion has in a circumferential direction at least section-wise a notch formed therein forming upon rotation of the pump rotor a pressure compensation bypass between the second expelling volume and the second suction volume.

Thus, the trochoidal medicament pump device may provide a reliable delivery of a medicament. Within the context of rotary pumps, a trochoid is defined as the plane curve that is generated by following the trajectory of a point fixed relative to one circle, where that circle rolls along another fixed circle. In the literature, this is also sometimes described as an epitrochoid or hypotrochoid, depending on whether the rolling circle contacts externally or internally with the fixed circle respectively. Thus, a trochoidal shape is one that has been generated using this geometrical method. The larger dimensions of the rotor in combination with the over-mold of an elastic or elastomeric material provide a reliable sealing over the pump chamber. It is the intention to make the pump chamber airtight, so that the pressure can get low enough to draw fluid up tubes and move, e.g., syringe/cartridge plungers, and to reduce any variability of dosage over time, even with any external influences. The rigid rotor core maintains the general geometry of the rotor and allows a reliable coupling of the rotor to the driving components, which drive the rotor over the pump body. Rigid means that the material is non-elastomeric, or at least less elastomeric than the elastomeric over-mold. The elastomeric over-mold provides a reliable sealing regardless in which rotational position the rotor is over the pump body. Upon eccentric rotation of the rotor, the rotor forms a suction chamber being in fluid communication with the inlet so that a fluid can enter the suction chamber. The eccentric rotation enlarges the suction volume upon further rotation so that a fluid is sucked into the suction volume. The suction volume rotates with the rotor until one of the apexes passes the inlet and thus closes the suction volume. As soon as the volume filled with fluid comes into communication with the outlet, the volume becomes an expelling volume and upon further rotation, the volume decreases and thus expels the fluid. At the same time, the following part of the triangular geometry of the rotor already operates as a suction volume and the previously described procedure repeats. Thus, the suction volume is the volume as long as it is connected to the inlet port. As soon as the volume is connected to the outlet, it becomes the expelling volume. The geometry of the trochoidal lobe in combination with the convex curved lines result in a very low volume after the expelling and before the next apex passes the outlet, so that almost all fluid or ideally all fluid is expelled. In some phases of the rotor rotation three portions of the rotor are in permanent contact with the inner wall of the first lobe, so that the rotor together with the inner wall always form a suction volume and an expelling volume at the same time, with the repeated procedure that the sucked fluid in the suction volume is transported until the volume becomes the expelling volume and the fluid is expelled, while the following geometry has just formed the following suction volume. The principle is analogue to the so called Wankel piston.

It should be noted that the terms apex and convex curved line is to be understood as seen from a top view, i.e., without the third dimension. In the third dimension, the apex is a line, and the convex curved line is a convex curved surface. This surface is limited by the two adjacent apex lines as well as both curved convex lines connecting the end points of both apex lines.

According to an embodiment, the first material class is a group consisting of liquid silicone rubber LSR, ethylene propylene diene monomer rubber EPDM, thermoplastic elastomer TPE), thermoplastic polyurethane (TPU), liquid crystalline polymer (LCP), butyl rubbers and nitrile rubbers.

Thus, flexible materials with adequate sealing properties, low friction over a pump body material and durability may be provided. It revealed that liquid silicone rubber LSR is particularly suitable for 2k molding especially in medical devices with the requirements of multiple sterilization processes not losing the physical properties. It revealed that ethylene propylene diene monomer rubber EPDM is particularly suitable for polar liquids. It revealed that TPE is particularly suitable for short time use, and has a larger compression set, has lower friction and a bonding compatibility with, e.g., a glass filled material. It revealed that TPU is particularly suitable for normal usage for tubes and bags and membranes. It revealed that butyl rubbers are suitable for cartridge plungers and nitrile rubbers for sealing elements.

According to an embodiment, the over-mold is made of liquid silicone rubber LSR with a hardness between 10 and 100 Shore (ShA), in particular between 50 and 100 Shore (ShA), in particular between 60 and 70 Shore (ShA).

Thus, a good balance may be achieved between reliable sealing an acceptable friction which is useful for operating the pump reliably with reasonable power effort. It revealed that using for the over-mold a LSR with a hardness between 50 and 100 Shore (ShA), and in particular between 60 and 70 Shore (ShA), this LSR configuration is suitable for fulfilling the requirements for low torque, minimal energy consumption, best fit in wear, friction, noise, sterilization, biocompatibility, lifetime usage and large temperature and humidity range usage.

According to an embodiment, the second material class is a group consisting of polyethylene terephthalate PET, polycarbonate PC, Tritan, cycloolefin copolymer COC, PBT, PPS, and PEI.

Thus, rigid materials with adequate sealing properties over the rotor material, low friction over a pump rotor material and durability may be provided. Tritan is a copolymer made from three monomers: dimethyl terephthalate (DMT), cyclohexanedimethanol (CHDM), and 2,2,4,4-tetramethyl-1,3-cyclobutanediol (CBDO). It revealed that polyethylene terephthalate PET is particularly suitable for long-term use and durability. It revealed that polycarbonate PC is particularly suitable for short storage time. It revealed that Tritan is particularly suitable for all kind of drugs and liquids with a long storage lifetime without quality loss. It revealed that COC (cycloolefin copolymer) is particularly suitable for most of the liquids and drugs. It revealed that PBT is comparable to PET for being particularly suitable for long-term use and durability. It revealed that PPS is particularly suitable for extreme temperature ranges with high accuracy, and PEI is particularly suitable for its high temperature resistance. In general, these materials are also suitable for essential requirement such as sterilization, biocompatibility, lifetime usages and large temperature and humidity range usage.

According to an embodiment, the outer dimensions of the pump rotor with the over-mold are larger than the corresponding inner dimensions of contacted portions of the pump chamber by a factor, which factor is between 1.01 and 1.1, in particular between 1.01 and 1.05, in particular between 1.02 and 1.03.

Thus, a reliable sealing can be established with a reasonable friction, which allows operation with reasonable power demand. It revealed that a factor between 1.01 and 1.1, in particular between 1.01 and 1.05, in particular between 1.02 and 1.03 is suitable for liquid viscosities up to 50 cP (centipoise).

According to an embodiment, the factor in the radial direction is between 1.01 and 1.1, in particular between 1.01 and 1.05, in particular between 1.02 and 1.03.

Thus, a reliable sealing in the radial direction can be achieved. Radial direction is the direction orthogonal to the rotational axis, which corresponds to the direction orthogonal of the apex lines abutting the wall portions of the pump chamber. It revealed that a factor between 1.01 and 1.1, in particular between 1.01 and 1.05, in particular between 1.02 and 1.03 is suitable for drugs between viscosities from 1 cP to 50 cP and chamber pressures up to 10 bar (1MPa).

According to an embodiment, the factor in the axial direction is between 1.01 and 1.1 in particular between 1.01 and 1.05, in particular between 1.01 and 1.03.

Thus, a reliable sealing in the axial direction can be achieved. Axial direction is the direction parallel to the rotational axis, which corresponds to the direction parallel to the apex lines abutting the wall portions of the pump chamber, which corresponds to the direction orthogonal of the bended lines abutting the inner wall of the cover and bottom of the pump chamber.

According to an embodiment, the outer dimensions of the pump rotor with the over-mold in radial direction are at least 0.01 mm larger than the corresponding inner dimensions of contacted portions of the pump chamber.

Thus, a reliable seal under applied pressure may be achieved in radial direction.

According to an embodiment, the outer dimensions of the pump rotor with the over-mold in axial direction are at least 0.07 mm larger than the corresponding inner dimensions of contacted portions of the pump chamber.

Thus, a reliable seal under applied pressure may be achieved in axial direction.

According to the invention as defined in independent claim 1 the pump chamber has a trochoidal shape with said first lobe and a second lobe forming a second inner wall portion of a pump volume of the pump chamber with the pump rotor eccentrically rotating with the apexes/apex lines traveling along a trochoidal line corresponding to the trochoidal shape of the pump chamber, thus forming upon rotation a second suction volume and a second expelling volume.

Thus, a symmetrical buildup can be provided which avoids unbalance when operating the pump device and allows a proper sealing over the environment in order to avoid impurities.

According to the invention as defined in independent claim 1 the second inner wall portion has in a circumferential direction at least section-wise a notch formed therein forming upon rotation of the pump rotor a pressure compensation bypass between the second expelling volume and the second suction volume.

Thus, upon use of only one lobe for pumping, the pressure build-up and under-pressure may be compensated. The purpose of the bypass in the second chamber area is to reduce the drive torque, and sealing defects driven by the generated air pressure in the second chamber. As the pressure upon rotation in the second suction chamber and the second expelling chamber are compensated, no second inlet and no second outlet have to be provided for the pressure compensation. Thus, the pump chamber may be sealed over the environment, which reduced impurities from outside.

According to an embodiment, the pump chamber in the pump volume of the second lobe has a second inlet in fluid communication with the in-port and a second outlet in fluid communication with the out-port with the pump rotor eccentrically rotating so that the three apexes of the rotor are in permanent sealing contact with the first inner wall portion of the first lobe and the second inner wall portion of the second lobe thus forming upon rotation a second suction volume in fluid communication to the second inlet and a second expelling volume in fluid communication to the second outlet.

Thus, a symmetrical pump geometry can be provided which provides two parallel operating pump sequences, one on the first lobe and the other on the second lobe. As the rotor is triangular and the pump chamber provides two lobes, the phase shift between the sucking an expelling characteristic of the first lobe is 180° over the sucking an expelling characteristic of the second lobe. When coupling both pump sequences together, the one thereof has a maximum expelling when the outlet is in the middle between two apexes, while the other one thereof has a minimum expelling when the third apex passes the outlet. A check valve at the outlet of each expelling chamber may avoid reflow of fluid into the expelling chamber. The same may apply for an inlet, and a check valve at the inlet of each suction chamber may avoid flow of fluid out of the suction chamber. It should be noted that the joint between the first lobe and the second lobe, i.e., where the first inner wall portion and the second inner wall portion transit into each other, is to be considered as belonging to both, the first lobe and the second lobe, and to both, the first inner wall portion and the second inner wall portion. It should also be noted, that in particular phases of the rotor rotation at least three distinct portions of the rotor are in permanent sealing contact for the first lobe and the at least three distinct portions of the rotor are in permanent sealing contact for the second lobe, which may share a portion, which may be a contact of the convex curved surface with the transit from the first lobe to the second lobe.

According to an embodiment, the second inlet is in fluid communication with the in-port and the second outlet is in fluid communication with the out-port.

Thus, not only first inlet, but also the second inlet can be connected to a common in-port, and also both, the first and second outlet is in fluid communication with the out-port so that the trochoidal medicament pump device can be operated with both lobes in parallel while having a single in-port and a single out-port, wherein the coupling of the parallel operated lobes and their inlets and outlets is established internally.

According to an embodiment, there is provided a giving set for providing a medicament from a reservoir to a patient, the giving set comprises a trochoidal medicament pump as described above, a first fluid conduit being with one end fixedly connected to the out-port and with the other end fixedly connected to a Luer-Lock.

Thus, the giving set can be provided with a medicament pump device without the need for additional joints between the pump device and the conduits. This may avoid impurities and keeps the possible number of connecting joints low. Further, it can be avoided that the medical pump device is unintentionally re-used, as the giving set definitively will not be re-used and disposed and so also the medical pump device. This makes the entire operation of the giving set safer.

According to an embodiment the first fluid conduit has an upstream section and a flexible downstream section, wherein the flexible downstream section is more flexible than the upstream section.

Thus, the giving set may stay more reliable connected to the patient. More flexible means that the more flexible section has a lower bending moment, in that the force required to bend to a certain extend along a given length is lower, in other words, more stable regarding bending. The flexible downstream section close to the patient allows compensation of a movement of the medicament pump device and unintentional separation of the giving set from the patient.

According to an embodiment, the first fluid conduit is made of a flexible tube of substantially constant diameter and wall thickness, wherein the flexible downstream section is formed by a pre-formed helical winding of the flexible tube, the geometry thereof providing higher flexibility than a non-helical portion.

Thus, it is possible to not only compensate a lateral, i.e., orthogonal to the extension of the conduit, movement of the medical pump device over the patient, but also a longitudinal, i.e., along the extension of the conduit, movement of the medical pump device over the patient. It should be noted that this may also include an embodiment, which has for the upstream portion a helical winding with low diameter windings, i.e., lower flexibility, and for the downstream portion a helical winding with large diameter windings, i.e., a higher flexibility.

According to an embodiment the giving set further comprises a second fluid conduit being with one end fixedly connected to the in-port and with the other end fixedly connected to a connector being adapted for being releasable connected to a fluid reservoir.

Thus, the giving set can be provided with a medicament pump device without the need for additional joints between the pump device and the conduits to the reservoir. This may avoid impurities and keeps the possible number of connecting joints low. Further, it can be avoided that the medical pump device is unintentionally re-used, as the giving set definitively will not be re-used and disposed and so also the medical pump device. This makes the entire operation of the giving set safer. It should be noted that the giving set may also a fixedly connected dripping volume between the reservoir and the medicament pump device.

According to an embodiment, the giving set further comprises a second fluid conduit being with one end fixedly connected to the in-port and with the other end fixedly connected to a pre-filled fluid reservoir.

Thus, a safe given set may be provided, which has a minimal number of connections and possible entering points for impurities. The medicament pump device may be adapted to the fluid in the pre-filled reservoir, so that the viscosity of the fluid matches the design of the medicament pump device. Any re-use can be avoided, which is of particular relevance for sensitive medicaments and critical diseases. The prefilled reservoir may include a predefined composition of medicament. A misalignment of medicament, in particular fluid and pump may be avoided.

According to an embodiment, the prefilled reservoir is gas volume free prefilled with a liquid.

Thus, the suction of gas can be avoided, and it can be guaranteed that regardless of the filling level of the reservoir always a fluid is delivered upon operation. As the trochoidal medicament pump device due to its construction is able to also suck against a certain height, the reservoir can also be disposed lower than the trochoidal medicament pump device. This makes it much easier to handle for a patient, in particular if the medicament pump device and the reservoir are used as a mobile delivery system which is carried by a patient and allows moving around.

According to an embodiment the giving set further comprises a tag representative for the liquid in the pre-filled reservoir and being adapted to be read out by a tag reader of a medicament pump driving device.

Thus, the operation of the trochoidal medicament pump device may be adapted to the type of fluid or liquid in the reservoir. Some liquids may have particular properties, which allow a defined driving dynamic, which can be setup based on the read-out tag, so that not only the medicament pump may be adapted to the liquid, but also the operation, e.g., the revolutions or change rate of revolutions, of the medicament pump device. This may of particular relevance if the liquid is a or includes a non-Newtonian fluid.

### Brief Description of the Figures

The invention will be described by way of the following drawings:
- Figure 1:: illustrates a schematic build-up in a top view of a trochoidal medicament pump device according to an exemplary embodiment not within the scope of independent claim 1.
- Figure 2:: illustrates a rotor core for a pump rotor for use in a trochoidal medicament pump device according to an exemplary embodiment.
- Figure 3:: illustrates a pump rotor with an over-molded core for use in a trochoidal medicament pump device according to an exemplary embodiment.
- Figure 4:: illustrates a top view of a trochoidal medicament pump device with removed cover according to an exemplary embodiment.
- Figure 5:: illustrates an exploded view of a trochoidal medicament pump device without cover according to another exemplary embodiment.
- Figure 6:: illustrates a schematic build-up in a top view of a trochoidal medicament pump device according to another exemplary embodiment.
- Figure 7:: illustrates giving set with a connected liquid/fluid reservoir and a trochoidal medicament pump device according to an exemplary embodiment.
- Figure 8:: illustrates giving set with a connected liquid/fluid reservoir and a trochoidal medicament pump device connected to driving device according to an exemplary embodiment.
- Figure 9:: illustrates giving set with a fluid conduit to be connected to a patient and a fluid conduit to be connected to a liquid reservoir according to an exemplary embodiment.

It should be noted that same or similar reference numerals illustrate same or similar components. Along these Figures exemplary embodiments of the invention will be described as follows.

### Detailed Description of Exemplary Embodiments

The invention will be described along exemplary embodiments, which are illustrated in the above references figures and will be described in detail in the following.

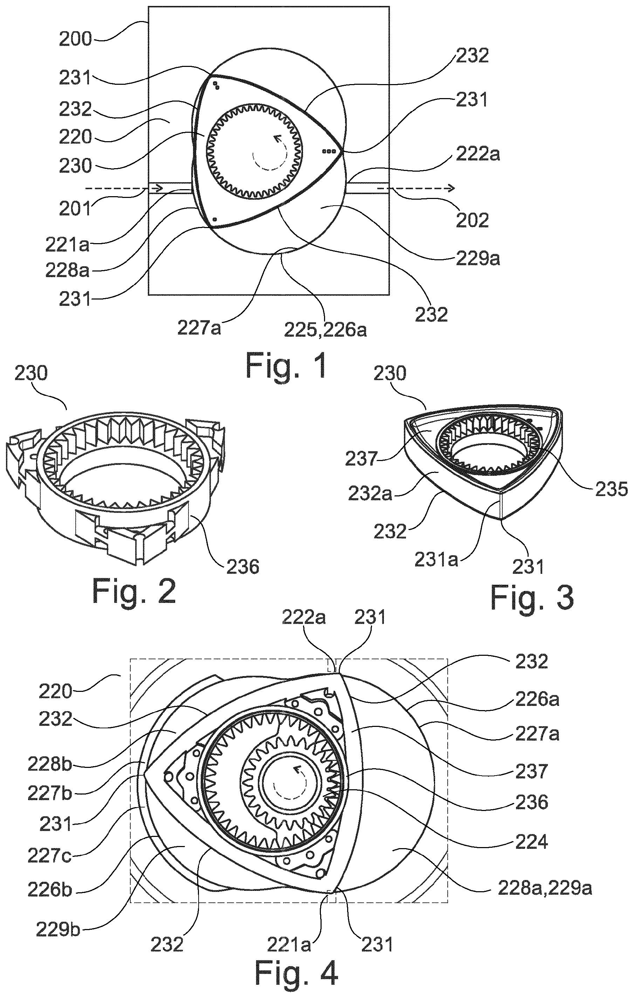

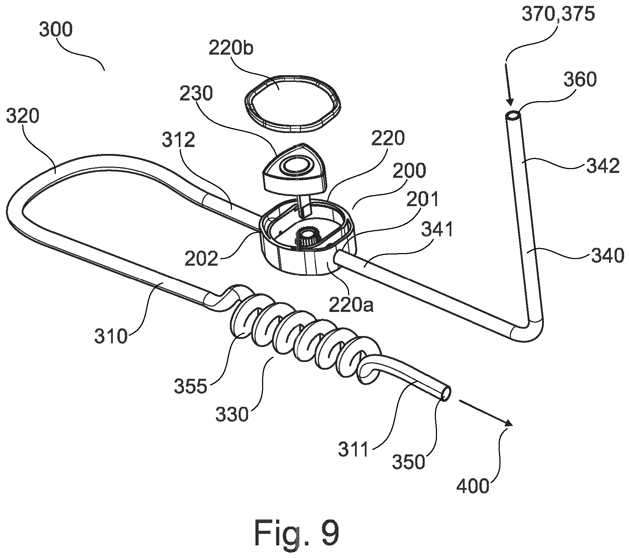

Figure 1 illustrates a schematic build-up in a top view of a trochoidal medicament pump device according to an exemplary embodiment not within the scope of independent claim 1. The trochoidal medicament pump device 200 has an in-port 201 and an out-port 202. The in-port receives the fluid or liquid to be pumped and the out-port delivers the fluid or liquid to be delivered. The in-port and the out-port may have releasable couplings or may be fixedly connected to conduits or the like. The trochoidal medicament pump device 200 has a pump body 220, which may have a housing 220a and a cover 220b. The housing 220a and the cover 220b, which is illustrated in Figure 9, define within the pump body 220 a pump chamber 225. The pump chamber 225 has rotating therein a pump rotor 230. The pump rotor 230 is of a triangular shape when seen from top. Seen from top the triangular shape has three apexes 231 and between each two apexes a convex curved line 232. When seen as a three-dimensional object, the apexes 231 are apex lines 231a extending parallel to the rotational axis of the pump rotor 230, and the convex curved lines 232 are convex curved surfaces 232a, extending between the apex lines 231a. The apex lines 231a and the convex curved lines 232a are in contact with wall portions 227a, 227b of the pump chamber 225. The apex lines 231a are always in contact with one of the wall portions 227a, 227b. The pump chamber 225 has two lobes 226a, 226b. Within the pump chamber 225 the pump rotor 230 rotates, so that the contact between the pump rotor 230, in particular its apex lines 231a and also the convex curved surfaces 232a, and the wall sections 227a, 227b of the lobes 226a, 226b form chambers, which rotate with the eccentrically rotating pump rotor 230 and successively are brought into communication with an inlet 221a and an outlet 222a. The apexes 231 of the eccentrically rotating pump rotor 230 trace a trochoidal line corresponding to a trochoidal shape that is equivalent or larger than the corresponding inner dimensions of the pump chamber 225. When in connection with the, or one of the inlets 221a, the chamber operates as a suction chamber 228a. As the chamber upon rotation successively enlarges its volume, the chamber sucks a fluid or liquid from the in-port 201. Upon rotation, the suction chamber rotates until it leaves the inlet 221a. Afterwards, the chamber gets into communication with an outlet 222a and becomes an expelling chamber 229a. Upon further rotation, the expelling chamber 229a successively decreases its volume and thus expels the fluid through the output 222a until the volume arrives at its minimum, which may be close to zero. At the same time, the following geometry of the rotor 230 proceeds likewise, so that the next chamber becomes the expelling chamber 229a and expels the next fluid amount upon rotation of the pump rotor 230.

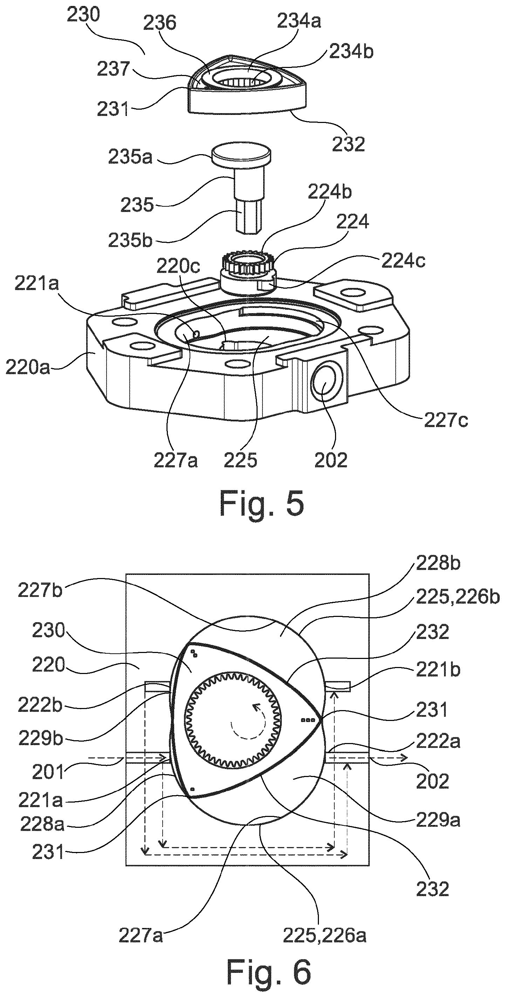

Figure 2: illustrates a rotor core 236 for a pump rotor 230 for use in a trochoidal medicament pump device 200 according to an exemplary embodiment. The rotor core forms a stable structure and provides the rigid geometry for driving the pump rotor 230 and to keep it in the correct position and trajectory upon eccentric rotation. For this purpose, the pump rotor 230, in particular the rigid core 236 has a sliding or guiding surface 234a on the pump rotor 230 and in particular the rigid rotor core 236 of the pump rotor 230, which receives an eccentrically traveling disk 235a of a driving pinion 235, which eccentrically rotation disk 235a forces the pump rotor 230 onto an eccentric trajectory. The pump rotor 230, in particular its rigid core 236 has also a gear section 234b, which meshes with a gear section 224b of a gear wheel 224 on the pump body 220, in particular the housing 220a, and upon eccentric traveling, forces the pump rotor 230 to rotate. The meshing can be seen in Figure 4, where the pinion 235 and the eccentrically rotation disk 235a thereof being removed. The design of the driving pinion 235 can be seen in Figure 5.

Figure 3 illustrates a pump rotor with an over-molded core for use in a trochoidal medicament pump device according to an exemplary embodiment. The core 236 serves for a stable and defined geometry for driving the pump rotor 230. The elastic or elastomeric over-mold 237 serves for sealing the pump rotor 230 over the pump chamber 225, in particular for sealing over the wall section 227a, 227b and the bottom of the housing 220a and the cover 220b of the housing. The apex lines 231a seal over the wall sections 227a, 227b, whereas the convex curved lines 232 seal over the bottom of the housing 220a and the cover 220b, as illustrated in Figure 5. The pump rotor 230 with the core 236 and the over-mold 237 may be manufactured in a 2K- manufacturing process. The core may be made of e.g., reinforced Polyetheretherketon PEEK or reinforced Polyethylene terephthalate PET. The core may also be made from other materials being sufficiently rigid. The aforementioned materials for the core revealed a good compatibility with the over-mold material. The over-mold 237 may be made of liquid silicone rubber LSR, ethylene propylene diene monomer rubber EPDM, thermoplastic elastomer TPE, thermoplastic polyurethane TPU, butyl rubbers and nitrile rubbers. The aforementioned materials have a good compatibility with the selected materials of the core 236, but also with the materials of the pump body 220, in particular the housing 220a and the cover 220b. The compatibility with the pump body 220 includes a compatibility with respect to resistivity over medicaments and compatibility with the viscosity of the medicaments, as well as friction between the over-mold 237 and the materials of the pump body 225. It should be noted that this also applies for a pump body 225, which has a two or more material composition with particular materials for the pump body 225 and other materials for the wall sections 227a, 227b, the bottom and the cover 220b thereof, in other words those parts of the body which are in contact with the pump rotor 230, in particular its over-mold 237.

Figure 4 illustrates a top view of a trochoidal medicament pump device 200 with removed cover according to an exemplary embodiment. Figure 4 illustrates the trochoidal medicament pump device with removed cover 220b and with removed driving pinion 235. Upon rotation of the pump rotor 230 in a planetary gear manner around the gear wheel 224, which is fixed to the pump body 220, the apexes 231 of the pump rotor 230 follow the trochoidal shape of the pump chamber 225, in particular its wall portion 227a on the right side in Figure 4. The apex lines 231a seal the pump rotor 230 over the inner wall portions and this form the suction chamber 228a and the expelling chamber 229a, respectively. The fluid to be pumped and delivered enters the pump chamber 225 over the inlet 221a and leaves the pump chamber 225 over the outlet 222a. The illustrated situation in Figure 4 is the transit where the suction chamber 228a becomes an expelling chamber 229a. It can be seen, that one of the apexes 231 has just passed the inlet 221a and thus closed the chamber and terminated the suction phase, before the ahead traveling apex 231 has not yet passed the outlet 222a and thus not yet opened the chamber for expelling the fluid. It should be noted that the apexes 231 also follow the wall sections 227b on the other side, the left side in Figure 4. However, in the illustrated embodiment of Figure 4, which corresponds to the embodiment illustrated in Figure 1, only one inlet 221a and only one outlet 222a are provided on the right lobe 226a. The other lobe 226b on the left side does not have an inlet and an outlet, so that in the respective the suction chamber 228b on the left side, a vacuum arises upon rotation. At the same time in the expelling chamber 229b on the left side the pressure arises upon rotation. As the vacuum in chamber 228b and the over pressure in chamber 229b lead to increased friction and load of the rotor 230, this vacuum and pressure, respectively should be avoided. As a further inlet and outlet should be avoided in case they are not used for pump and delivery, the pressure difference is compensated through a notch 227c, which follows along a particular section of the wall section 227b on the left side in Figure 4. The notch bypasses the sealing of the apex 231 at the inner wall portion 227b and allows the overpressure from the expelling chamber to transit and compensate the vacuum in the suction chamber 228b. Thus, the pressure difference can be compensated, and the friction and load can be reduced. The notch may be formed by a chamfer as illustrated in Figure 4. The chamfer may have rounded edges at least at the side where the apex line is in contact with the edge of the chamfer. The notch may also be realized by a groove along the inner wall portion 227b or even by a bypassing channel having one opening in the area of the suction chamber 228b and the other opening in the area of the expelling chamber 229b.

Figure 5 illustrates an exploded view of a trochoidal medicament pump device without cover 220b according to another exemplary embodiment. The position of the cover 220b is illustrated in Figure 9. It should be noted that the traveling trajectory of the pump rotor 230 is defined by the eccentric disk 235a, which is illustrated in Figure 5, and which is omitted in Figure 4 for illustrative purposes. The eccentrically rotating disk 235a upon centric rotation of the pinion 235 and its drive coupling 235b forces the pump rotor 230 onto an eccentric path. The eccentric disk 235a is rotationally bedded over a corresponding bearing surface 234a on the rotor 230, in particular its core 236. As during eccentric rotation, the gear section 234b of the pump rotor 230 remains in engagement with the gear section 224b of gear wheel 224 of the pump body, the pump rotor 230 not only eccentrically travels along an eccentric path, but also rotates, so that the apexes follow the trochoidal path of the first and second lobe 226a, 226b and the corresponding wall portions 227a, 227b. As can be seen in Figure 5, the gear wheel 224 is provided as a separate element which however has a protrusion 224c which engages a detention 220c in the pump body so that the gear wheel 224 is fixed over the pump body. The fixation can also be realized by other measures, like adhesives, positive fit connections or by providing the gear wheel 224 in one piece with the pump body 225. With respect to the remaining elements in Figure 5 it is referred to the above-described Figures.

The pump rotor 230 has an over-mold 237, which has with respect to the contacting portions to the wall portions 227a, 227b of the pump chamber 225 a larger dimension than the pump chamber, so that the elastic or elastomeric over-mold is compressed and provides a reliable sealing. These contacting portions are the apex lines 231a of the rotor 230 and also portions of the convex curved surfaces 232a, which are in contact to the inner wall portions 227a, 227b, in particular with the transit from the first wall portion 227a of the first lobe 226a to the second wall portion 227b of the second lobe 226b. The elastic or elastomeric material of the over-mold 237 may be selected out of a first material class, the class may be a group consisting of liquid silicone rubber LSR, ethylene propylene diene monomer rubber EPDM, thermoplastic elastomer TPE, thermoplastic polyurethane TPU butyl rubbers and nitrile rubbers. In particular, if the over-mold 237 is made of liquid silicone rubber LSR, the LSR is designed to have a hardness between 10 and 100 Shore (ShA), in particular between 50 and 100 Shore (ShA), in particular between 60 and 70 Shore (ShA). The hardness in some instances depend on the expected pressure of the fluid and of the viscosity of the fluid to be pumped. As an alternative, the over-mold may be made of a medical grade TPE (thermoplastic elastomer), and the TPE over-mold 237 is designed to have a hardness between 29 and 90 Shore (ShA), in particular between 40 and 80 Shore (ShA), in particular between 50 and 70 Shore (ShA). As a further alternative, the over-mold may be made of a TPU (thermoplastic Polyurethane), and the TPU over-mold 237 is designed to have a hardness between 29 and 90 Shore (ShA), in particular between 40 and 80 Shore (ShA), in particular between 50 and 70 Shore (ShA). The hardness in some instances depend on the expected pressure of the fluid and of the viscosity of the fluid to be pumped

It revealed that a considerable number of infusions are based on fluids/liquids having a viscosity, which is similar to the viscosity of water, which is for water at 20°C about 1cP at a flow of 500ml/h. When using a small canula, e.g., that of a 31G needle with an inner diameter of 0.11 mm, an exemplary inner pressure within an exemplary pump chamber is between 6 and 7 bar.

The hardness may also depend on the material used for the pump body 225, in particular for the inner wall sections 227a, 227b, the bottom and the cover 220b of the pump body 220. These sections may be manufactured of a material of a second class of material, wherein the second material class is a group consisting of polyethylene terephthalate PET, polycarbonate PC, Tritan, high density polyethylene HDPE, cycloolefin copolymer COC, PBT, PPS, and PEI. The inner dimensions of the pump chamber 225, in particular the inner wall portions 227a, 227b, and the outer dimensions of the pump rotor 230, in particular its over-mold 237 may also depend on the expected pressure and the expected viscosity of the fluid. The outer dimensions of the pump rotor 230 with the over-mold 237 may be larger than the corresponding inner dimensions of contacted portions of the pump chamber 225 by a factor, which factor is between 1.01 and 1.1, in particular between 1.01 and 1.05, in particular between 1.01 and 1.03. This means the oversize by this factor has to be compensated by the elastic or elastomeric material of the over-mold 237. As the over-mold has a smaller dimension than the entire rotor 230, and the core 236 is not or only less elastic or elastomeric, the elastic or elastomeric material of the over-mold needs to be more elastic or elastomeric than by only the above-mentioned factor. The elasticity may occur in different directions, i.e. in radial direction and in axial direction. The reason is that the acting frictional forces have different directions, and the sealing along the apex line 231a is shorter than the convex curved sealing line 232. Further, the movement of the rotor 230 results in a traverse force onto the apex line 231a, whereas the force onto the convex curved lines 232 is at least partially along the extension of the convey curved sealing line. Therefore, the factor in the radial direction may be between 1.01 and 1.1 (i.e., percentage increase 1-10%), in particular between 1.01 and 1.05 (i.e., percentage increase 1-5%), in particular between 1.02 and 1.03 (i.e., percentage increase 2-3%) whereas the factor in the axial direction may be between 1.01 and 1.1 (i.e., percentage increase 1-10%), in particular between 1.01 and 1.05 (i.e., percentage increase 1-5%), in particular between 1.01 and 1.03 (i.e., percentage increase 1-3%). The relation of the percentage increase of the radial factor and the percentage increase of the axial factor (percentage increase axial factor divided by the percentage increase radial factor) may be between 1 and 10 (i.e., between 1%/1% and 10%/1%), in particular between 1 and 5 (i.e., between 1%/1% and 5%/1%), in particular between 2 and 3 (i.e., between 2%/1% and 3%/1%).

Figure 6 illustrates a schematic build-up in a top view of a trochoidal medicament pump device according to another exemplary embodiment. The trochoidal medicament pump device 200 as illustrated in Figure 6 is similar to the device illustrated in Figure 1. In addition to what is described with respect to Figure 1, the trochoidal medicament pump device 200 of Figure 6 has not only an inlet 221a and an outlet 222a in the first lobe 226a, but also has a further inlet 221b and a further outlet 222b in the second lobe 226b. This allows using also the second lobe as a pump, which may be operated in parallel to the pump formed in the first lobe 226a. The first inlet 221a and the second inlet 221b both may be in fluid communication with the in-port 201. At the same time the first outlet 222a and the second outlet 222b may be in fluid communication with the out-port 202. Thus, both pump paths can be coupled via a common in-port 201 and a common out-port 202. The in-port 201 receives the fluid or liquid to be pumped and the out-port 202 delivers the fluid or liquid to be delivered. The in-port and the out-port may have releasable couplings or may be fixedly connected to conduits or the like. The triangular pump rotor 230 travels as describe above. The apex lines 231a and the convex curved lines 232a are in contact with wall portions 227a, 227b of the pump chamber 225. The apex lines 231a are always in contact with one of the wall portions 227a, 227b. The pump chamber 225 has two lobes 226a, 226b. Within the pump chamber 225 the pump rotor 230 rotates, so that the contact between the pump rotor 230, in particular its apex lines 231a and also the convex curved surfaces 232a, and the wall sections 227a, 227b of the lobes 226a, 226b form chambers, which rotate with the eccentrically rotating pump rotor 230 and successively are brought into communication with the first inlet 221a, the first outlet 222a. the second inlet 221b and the second outlet 222b. When in connection with the respective inlet 221a, 221b, the chamber operates as a suction chamber 228a, 228b. As the chamber upon rotation successively enlarges its volume, the chamber sucks a fluid or liquid from the in-port 201. Upon rotation, the suction chamber rotates until it leaves the respective inlet 221a, 221b. Afterwards, the chamber gets into communication with the respective outlet 222a, 222b and becomes an expelling chamber 229a, 229b. Upon further rotation, the expelling chamber 229a, 229b successively decreases its volume and thus expels the fluid through the output 222a, 222b until the volume arrives at its minimum, which may be close to zero. At the same time, the following geometry of the rotor 230 proceeds likewise, so that the next chamber becomes the expelling chamber 229a, 229b and expels the next fluid amount upon rotation of the pump rotor 230. The in-flow follows in Figure 6 the dashed lines and arrows, while the out flow follows the dashed-dotted lines in Figure 6.

As can be seen from Figure 6, the first suction chamber 228a is at a minimum volume starting the suction process, while the second suction chamber 228b is at a maximum volume having almost finished the suction process. Likewise, the first expelling chamber 229a is at a maximum volume starting the expelling process, while the second expelling chamber 229b is at a minimum volume having almost finished the expelling process. As the expelling process is not linear and has a break while an apex crosses the respective output, a single pump has a non-continuous feeding upon continuous rotation of the pump rotor 230. However, as the expelling process of the pump in the first lobe 226a is phase shifted over the expelling process in the second lobe 226b, the interruption may be compensated. So that no interruption occurs at the out-port 202. In order to avoid unintended backflow of fluid into a chamber, check valves or flow resistors (not illustrated in Figure 6) may be provided which avoid or reduce unintended back flow.

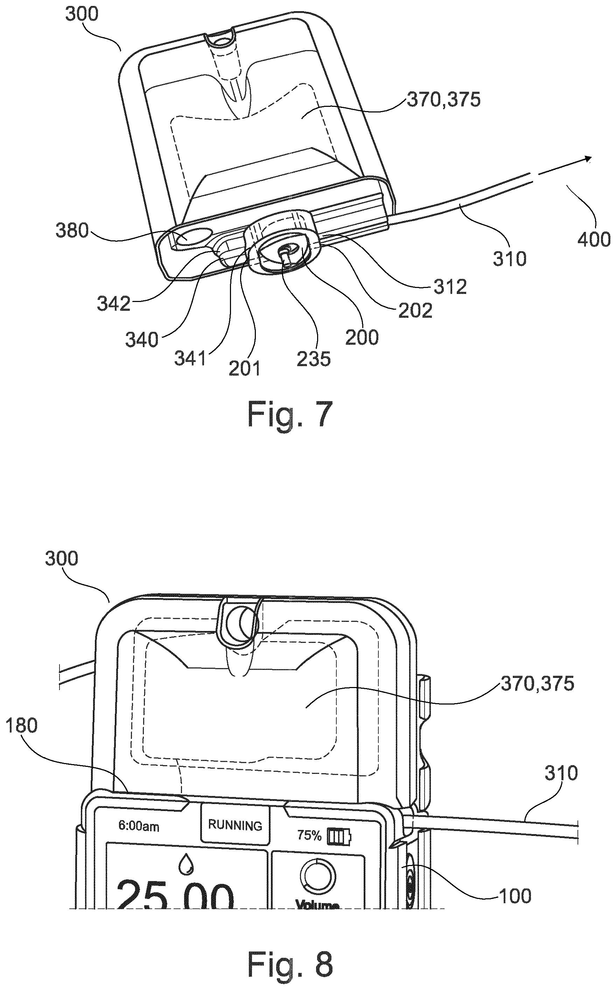

Figure 7 illustrates a giving set with a connected liquid/fluid reservoir and a trochoidal medicament pump device according to an exemplary embodiment. The trochoidal medicament pump device is as described above with respect to Figures 1 to 6. A giving set is a device which is used for applying a medicament to a patient. Conventional giving sets are known and may include a connection to a reservoir and a connection to a patient and dosing facilities for setting a flow rate. The giving set 300 includes a trochoidal medicament pump device 200 and further has a first fluid conduit 310 for connecting the trochoidal medicament pump device 200, in particular its out-port 202 to a patient 400. Likewise, the giving set 300 has a second fluid conduit 340 for connecting the trochoidal medicament pump device 200, in particular its in-port 201 with a reservoir 370. The giving set 300 illustrated in Figure 7 has the reservoir 370 fixedly connected to the second fluid conduit 340, The first end 341 of the second conduit 340 is fixedly connected to the in-port 201 of the trochoidal medicament pump device 200 and the second end 342 of the second conduit 340 is fixedly connected to the reservoir 370. This reduces joining connections and impurities in the system. The reservoir 370 may be a pre-filled reservoir 375, so that the giving set is ready for use in combination with a trochoidal medicament pump device 200. The first conduit 310 with its first end can be connected to the patient 400, and with its second end 312 is fixedly connected to the out-port 202 of the trochoidal medicament pump device 200. The trochoidal medicament pump device 200 may be adapted to the fluid in the prefilled reservoir 375. As the reservoir usually is not refilled and disposed, also the trochoidal medicament pump device 200 is disposed and an unintended re-use can be avoided. The reservoir may be a flexible volume like a bag and with the fluid therein may be gas free, so that irrespective of the position of the reservoir and its outlets, a fluid can be taken from the reservoir. As the trochoidal medicament pump device 200 is able to suck, the reservoir may even be disposed lower than the trochoidal medicament pump device 200, i.e., with the trochoidal medicament pump device 200 on top of the reservoir 370, 375. The giving set 300 may have a readable tag 380 carrying information representative for the giving set, e.g., with respect to the tape of fluid, the volume of the reservoir, the type of the trochoidal medicament pump device 200, etc. This tag can read by a tag reader 180 on e.g., a driving device 100 for the trochoidal medicament pump device 200, as will be described with respect to Figure 8. The tag 380 may be an RFID device, or a machine-readable optical tag, but may also be an actively sending tag. The tag may also include a certain intelligence and may be connected to sensors or the like, which sense parameters on or of the giving set, so that this information can be provided upon reading the tag. The trochoidal medicament pump device 200 has a coupling to be driven by a driving device 100. The coupling may be realized by a coupling portion 235 of a driving pinion, as describes above, and may be coupled to the driving device 100 by the coupling portion 235.

Figure 8 illustrates a giving set with a connected liquid/fluid reservoir and a trochoidal medicament pump device connected to driving device according to an exemplary embodiment. The giving set 300 is coupled via the above-described driving coupling 235 to the driving unit 100. The driving unit may also have a tag reading facility 180 for reading the tag 380 or communicating with the tag 380. The trochoidal medicament pump device 200 in this embodiment is received in aby of the driving unit in order to protect the trochoidal medicament pump device 200, its ports 201, 202 and also the conduits 310, 340. The giving set 300 may be locked with respect to the driving unit 100 in order to avoid unintended separation of the reservoir 370, 375 and the trochoidal medicament pump device 200 from the driving unit.

Figure 9 illustrates giving set with a fluid conduit to be connected to a patient and a fluid conduit to be connected to a liquid reservoir according to an exemplary embodiment. Figure 9 illustrates the implementation of a trochoidal medicament pump device as described above with respect to Figures 1 to 6. The giving set 300 includes the trochoidal medicament pump device 200 and further has a first fluid conduit 310 for connecting the trochoidal medicament pump device 200, in particular its out-port 202 to a patient 400. Likewise, the giving set 300 has a second fluid conduit 340 for connecting the trochoidal medicament pump device 200. For this purpose, the second conduit 340 has a first end 341, which is connected to the trochoidal medicament pump device 200, in particular its in-port 201. A second end 342 of the second conduit 340 may be connected to a reservoir 370, 375. This can be done via a connector 360. The reservoir 370, 375 may also be fixedly connected as described with respect to figure 7 above. The giving set illustrated in Figure 9 for the first conduit 310 has an upstream section 320 and a downstream section 330. In the upstream section 320 the second end 312 of the first conduit 310 is fixedly connected to the out-port 202 of the trochoidal medicament pump device 200. In the downstream section 330 the first end 311 of the first conduit 310 is connectable to a patient 400, e.g., via a Luer lock device 350. The downstream section 330 in this embodiment has a higher flexibility than the upstream section, which means that it can be easier bended. This is achieved in the illustrated embodiment by providing a helical winding 335, which elongates the flow length of the conduit and therefore allows higher flexibility, however without elongated dimensions, as the helical winding 335 is not longer than a comparable other section and does not increase the physical distance from the reservoir to the patient. The helical winding may not only compensate bending forces traverse to the longitudinal extension, but also forces along the longitudinal extension of the first conduit, as the helical winding may act like a spring.

### Reference numbers

- 100: driving unit for driving the trochoidal medicament pump device
- 180: tag reader on driving unit
- 200: trochoidal medicament pump device
- 201: in-port of trochoidal medicament pump device
- 202: out-port of trochoidal medicament pump device
- 220: pump body
- 220a: housing of pump body
- 220b: cover of pump body
- 220c: locking notch of pump housing / pump body
- 221a: first inlet of pump chamber
- 221b: second inlet of pump chamber
- 222a: first outlet of pump chamber
- 222b: second outlet of pump chamber
- 224: gear wheel
- 224b: gear section of gear wheel
- 224c: locking protrusion of gear wheel
- 225: pump chamber
- 226a: first lobe of pump chamber
- 226b: second lobe of pump chamber
- 227a: first inner wall portion
- 227b: second inner wall portion
- 227c: notch
- 228a: first suction volume
- 228b: second suction volume
- 229a: first expelling volume
- 229b: second expelling volume
- 230: pump rotor
- 231: apex
- 231a: apex line / apex sealing line of pump rotor
- 232: convex curved line /convex curved sealing line of pump rotor
- 232a: convex curved surface
- 234a: sliding / guiding surface on pump rotor / rigid rotor core of pump rotor
- 234b: gear section on pump rotor / rigid rotor core of pump rotor
- 235: drive pinion
- 235a: eccentric sliding disk of drive pinion
- 235b: drive coupling of drive pinion
- 236: (rigid) rotor core of pump rotor
- 237: (elastic or elastomeric) over-mold of pump rotor
- 300: giving set
- 310: first fluid conduit
- 311: first end of first fluid conduit
- 312: second end of first fluid conduit
- 320: upstream section of first fluid conduit
- 330: (flexible) downstream section of first fluid conduit
- 335: (flexible) helical winding of first fluid conduit
- 340: second fluid conduit
- 341: first end of second fluid conduit
- 342: second end of second fluid conduit
- 350: Luer-Lock (at (first) end of first fluid conduit)
- 360: connector at (second) end of second fluid conduit
- 370: fluid reservoir
- 375: pre-filled fluid reservoir
- 380: tag on (pre-filled) fluid reservoir
- 400: patient

## Claims

1. A trochoidal medicament pump device (200) comprising:
a pump body (220);
an in-port (201) for entering a medicament to be administered;
an out-port (202) for administering a medicament;
a pump rotor (230) having a triangular convex shape and having an eccentric trajectory upon rotation within the pump body;
wherein the pump rotor (230) has three equidistant apexes (231), each two thereof are connected with a convex curved line (232);
wherein the pump body (220) has a pump chamber (225);
wherein the pump chamber (225) has a first lobe (226a) forming a first inner wall portion (227a) of a pump volume of the pump chamber (225) with a first inlet (221a) in fluid communication with the in-port (201) and a first outlet (222a) in fluid communication with the out-port (202) with the pump rotor (230) eccentrically rotating so that the rotor with the first wall portion forms upon rotation a first suction volume (228a) in fluid communication to the first inlet (221a) and a first expelling volume (229a) in fluid communication to the first outlet (222a),
wherein the pump rotor (230) has a rigid rotor core (236) and an elastomeric over-mold (237) over at least sealing lines along the apexes and sealing lines along the convex curved lines,
wherein the over-mold is made of an elastomeric material of a first material class,
wherein the first inner wall portion (227a) is made of a material of a second material class,
**characterised in that**
the outer dimensions of the pump rotor (230) with the over-mold (237) are larger than the corresponding inner dimensions of contacted portions of the pump chamber (225),
wherein the apexes (231) of the eccentrically rotating pump rotor (230) trace a trochoidal line corresponding to a trochoidal shape that is equivalent or larger than the corresponding inner dimensions of the pump chamber (225)
wherein the pump chamber (225) has a trochoidal shape with said first lobe and a second lobe (226b) forming a second inner wall portion (227b) of a pump volume of the pump chamber (225) with the pump rotor (230) eccentrically rotating with the apexes traveling along a trochoidal line corresponding to the trochoidal shape, thus forming upon rotation a second suction volume (228b) and a second expelling volume (229b),
wherein the second inner wall portion (227b) has in a circumferential direction at least section-wise a notch (227c) formed therein forming upon rotation of the pump rotor a pressure compensation bypass between the second expelling volume (229b) and the second suction volume (228b).

2. The trochoidal medicament pump device of claim 1, wherein the first material class is a group consisting of liquid silicone rubber LSR, ethylene propylene diene monomer rubber EPDM, thermoplastic elastomer TPE, thermoplastic polyurethane PU butyl rubbers and nitrile rubbers.

3. The trochoidal medicament pump device of any of claims 1 and 2, wherein the over-mold is made of liquid silicone rubber LSR with a hardness between 10 and 100 Shore (ShA), in particular between 50 and 100 Shore (ShA), in particular between 60 and 70 Shore (ShA).

4. The trochoidal medicament pump device of any of claims 1 to 3, wherein the second material class is a group consisting of polyethylene terephthalate PET, polycarbonate PC, Tritan, high density polyethylene HDPE, COC (cycloolefin copolymer), PBT, PPS, and PEI.

5. The trochoidal medicament pump device of any of claims 1 to 4, wherein the outer dimensions of the pump rotor (230) with the over-mold are larger than the corresponding inner dimensions of contacted portions of the pump chamber (225) by a factor, which factor is between 1.01 and 1.1, in particular between 1.01 and 1.05, in particular between 1.02 and 1.03.

6. The trochoidal medicament pump device of claim 5, wherein the factor in the radial direction is between 1.01 and 1.1, in particular between 1.01 and 1.05, in particular between 1.02 and 1.03.

7. The trochoidal medicament pump device of any of claims 5 and 6, wherein the factor in the axial direction is between 1.01 and 1.1, in particular between 1.01 and 1.05, in particular between 1.01 and 1.03.

8. The trochoidal medicament pump device of any of claims 1 to 7, wherein the pump chamber (225) in the pump volume of the second lobe (226b) has a second inlet (221b) in fluid communication with the in-port (201) and a second outlet (222b) in fluid communication with the out-port (202) with the pump rotor (230) eccentrically rotating so that at least three distinct portions (231, 232) of the rotor (230) are in permanent sealing contact with the first inner wall portion (227a) of the first lobe (226a) and second inner wall portion (227b) of the second lobe (226b) thus forming upon rotation a second suction volume (228b) in fluid communication to the second inlet (221b) and a second expelling volume (229b) in fluid communication to the second outlet (222b), wherein in particular the second inlet (221b) is in fluid communication with the in-port (201) and the second outlet (222b) is in fluid communication with the out-port (202).

9. A giving set (300) for providing a medicament from a reservoir to a patient (400), the giving set (300) comprises:
a trochoidal medicament pump (200) according to any of claims 1 to 8;
a first fluid conduit (310) being with one end (312) fixedly connected to the out-port (202) and with the other end (311) fixedly connected to a Luer-lock (350).

10. The giving set of claim 9, wherein the first fluid conduit (310) has an upstream section (320) and a flexible downstream section (330), wherein the flexible downstream section (330) is more flexible than the upstream section (320), wherein in particular the first fluid conduit (310) is made of a flexible tube of substantially constant diameter and wall thickness, and the flexible downstream section (330) is formed by a pre-formed helical winding (335) of the flexible tube, the geometry thereof providing higher flexibility than a non-helical portion.

11. The giving set of any of claims 9 to 10, wherein the giving set (300) further comprises a second fluid conduit (340) being with one end (341) fixedly connected to the in-port (201) and with the other end (342) fixedly connected to a connector (360) being adapted for being releasable connected to a fluid reservoir (370).

12. The giving set of any of claims 9 to 11, wherein the giving set (300) further comprises a second fluid conduit (340) being with one end (341) fixedly connected to the in-port (201) and with the other end (342) fixedly connected to a pre-filled fluid reservoir (375), wherein in particular the prefilled reservoir (375) is gas volume free prefilled with a liquid.

13. The giving set of any of claims 9 to 12, wherein the giving set (300) further comprises a tag (380) representative for the liquid in the pre-filled reservoir (375) and being adapted to be read out by a tag reader (180) of a medicament pump driving device (100).

## Patentansprüche

1. Trochoidale Medikamentenpumpenvorrichtung (200), umfassend:
einen Pumpenkörper (220);
einen Einlass (201) zum Einbringen eines zu verabreichenden Medikaments;
einen Auslass (202) zur Verabreichung eines Medikaments;
einen Pumpenrotor (230) mit einer dreieckig-konvexen Form, der bei der Rotation innerhalb des Pumpenkörpers eine exzentrische Bahn beschreibt;
wobei der Pumpenrotor (230) drei gleichmäßig beabstandete Spitzen (231) aufweist, von denen jeweils zwei durch eine konvex gekrümmte Linie (232) miteinander verbunden sind;
wobei der Pumpenkörper (220) eine Pumpenkammer (225) aufweist; wobei die Pumpenkammer (225) einen ersten Lappen (226a) aufweist, der einen ersten inneren Wandabschnitt (227a) eines Pumpenvolumens der Pumpenkammer (225) bildet, mit einem ersten Einlass (221a), der mit dem Einlass (201) fluidisch verbunden ist, und einem ersten Auslass (222a), der mit dem Auslass (202) fluidisch verbunden ist, wobei der Pumpenrotor (230) exzentrisch rotiert, so dass der Rotor zusammen mit dem ersten Wandabschnitt bei der Rotation ein erstes Saugvolumen (228a) bildet, das mit dem ersten Einlass (221a) fluidisch verbunden ist, und ein erstes Ausstoßvolumen (229a), das mit dem ersten Auslass (222a) fluidisch verbunden ist; wobei der Pumpenrotor (230) einen starren Rotorkern (236) und eine elastomere Ummantelung (237) aufweist, die zumindest die Dichtlinien entlang der Spitzen sowie entlang der konvex gekrümmten Linien überdeckt,
wobei die Ummantelung aus einem elastomeren Material einer ersten Materialklasse besteht,
wobei der erste innere Wandabschnitt (227a) aus einem Material einer zweiten Materialklasse besteht,
**dadurch gekennzeichnet, dass** die Außenabmessungen des Pumpenrotors (230) mit der Ummantelung (237) größer sind als die entsprechenden Innenabmessungen der berührten Bereiche der Pumpenkammer (225),
wobei die Spitzen (231) des exzentrisch rotierenden Pumpenrotors (230) eine trochoidale Linie nachzeichnen, die einer trochoidalen Form entspricht, die gleich oder größer ist als die entsprechenden Innenabmessungen der Pumpenkammer (225),
wobei die Pumpenkammer (225) eine trochoidale Form mit dem genannten ersten Lappen und einem zweiten Lappen (226b) aufweist, der einen zweiten inneren Wandabschnitt (227b) eines Pumpenvolumens der Pumpenkammer (225) bildet, wobei der Pumpenrotor (230) exzentrisch rotiert und die Spitzen entlang einer trochoidalen Linie entsprechend der trochoidalen Form verlaufen, so dass bei der Rotation ein zweites Saugvolumen (228b) und ein zweites Ausstoßvolumen (229b) gebildet werden,
wobei der zweite innere Wandabschnitt (227b) in Umfangsrichtung zumindest abschnittsweise eine Nut (227c) aufweist, die bei der Rotation des Pumpenrotors einen Druckausgleichs-Bypass zwischen dem zweiten Ausstoßvolumen (229b) und dem zweiten Saugvolumen (228b) bildet.

2. Trochoidale Medikamentenpumpenvorrichtung (200) nach Anspruch 1, wobei die erste Materialklasse eine Gruppe ist, bestehend aus Flüssigsilikonkautschuk (LSR), Ethylen-Propylen-Dien-Kautschuk (EPDM), thermoplastischem Elastomer (TPE), thermoplastischem Polyurethan (PU), Butylkautschuken und Nitrilkautschuken.

3. Trochoidale Medikamentenpumpenvorrichtung (200) nach einem der Ansprüche 1 oder 2, wobei die Ummantelung aus Flüssigsilikonkautschuk (LSR) mit einer Härte zwischen 10 und 100 Shore (ShA), insbesondere zwischen 50 und 100 Shore (ShA), insbesondere zwischen 60 und 70 Shore (ShA), besteht.

4. Trochoidale Medikamentenpumpenvorrichtung (200) nach einem der Ansprüche 1 bis 3, wobei die zweite Materialklasse eine Gruppe ist, bestehend aus Polyethylenterephthalat (PET), Polycarbonat (PC), Tritan, hochdichtem Polyethylen (HDPE), COC (Cycloolefin-Copolymer), PBT, PPS und PEI.

5. Trochoidale Medikamentenpumpenvorrichtung (200) nach einem der Ansprüche 1 bis 4, wobei die Außenabmessungen des Pumpenrotors (230) mit der Ummantelung größer sind als die entsprechenden Innenabmessungen der berührten Bereiche der Pumpenkammer (225) um einen Faktor, wobei dieser Faktor zwischen 1,01 und 1,1 liegt, insbesondere zwischen 1,01 und 1,05, insbesondere zwischen 1,02 und 1,03.

6. Trochoidale Medikamentenpumpenvorrichtung (200) nach Anspruch 5, wobei der Faktor in radialer Richtung zwischen 1,01 und 1,1 liegt, insbesondere zwischen 1,01 und 1,05, insbesondere zwischen 1,02 und 1,03.

7. Trochoidale Medikamentenpumpenvorrichtung (200) nach einem der Ansprüche 5 oder 6, wobei der Faktor in axialer Richtung zwischen 1,01 und 1,1 liegt, insbesondere zwischen 1,01 und 1,05, insbesondere zwischen 1,01 und 1,03.

8. Trochoidale Medikamentenpumpenvorrichtung (200) nach einem der Ansprüche 1 bis 7, wobei die Pumpenkammer (225) im Pumpenvolumen des zweiten Lappen (226b) einen zweiten Einlass (221b) aufweist, der mit dem Einlass (201) fluidisch verbunden ist, und einen zweiten Auslass (222b), der mit dem Auslass (202) fluidisch verbunden ist, wobei der Pumpenrotor (230) exzentrisch rotiert, so dass mindestens drei verschiedene Bereiche (231, 232) des Rotors (230) in dauerhaftem Dichtkontakt mit dem ersten inneren Wandabschnitt (227a) des ersten Lappen (226a) und dem zweiten inneren Wandabschnitt (227b) des zweiten Lappen (226b) stehen, wodurch bei der Rotation ein zweites Saugvolumen (228b) gebildet wird, das mit dem zweiten Einlass (221b) fluidisch verbunden ist, und ein zweites Ausstoßvolumen (229b), das mit dem zweiten Auslass (222b) fluidisch verbunden ist, wobei insbesondere der zweite Einlass (221b) mit dem Einlass (201) und der zweite Auslass (222b) mit dem Auslass (202) fluidisch verbunden ist.

9. Verabreichungsset (300) zur Verabreichung eines Medikaments aus einem Reservoir an einen Patienten (400), wobei das Verabreichungsset (300) umfasst:
eine trochoidale Medikamentenpumpenvorrichtung (200) nach einem der Ansprüche 1 bis 8; eine erste Fluidleitung (310), die mit einem Ende (312) fest mit dem Auslass (202) verbunden ist und mit dem anderen Ende (311) fest mit einem Luer-Lock (350) verbunden ist.

10. Verabreichungsset (300) nach Anspruch 9, wobei die erste Fluidleitung (310) einen stromaufwärtigen Abschnitt (320) und einen flexiblen stromabwärtigen Abschnitt (330) aufweist, wobei der flexible stromabwärtigen Abschnitt (330) flexibler ist als der stromaufwärtigen Abschnitt (320), wobei insbesondere die erste Fluidleitung (310) aus einem flexiblen Schlauch mit im Wesentlichen konstantem Durchmesser und Wandstärke besteht und der flexible stromabwärtigen Abschnitt (330) durch eine vorgeformte, spiralförmige Wicklung (335) des flexiblen Schlauchs gebildet ist, deren Geometrie eine höhere Flexibilität als ein nichtspiralförmiger Abschnitt aufweist.

11. Verabreichungsset (300) nach einem der Ansprüche 9 bis 10, wobei das Verabreichungsset (300) ferner eine zweite Fluidleitung (340) umfasst, die mit einem Ende (341) fest mit dem Einlass (201) verbunden ist und mit dem anderen Ende (342) fest mit einem Anschlussstück (360) verbunden ist, das zum lösbaren Anschluss an ein Flüssigkeitsreservoir (370) ausgelegt ist.

12. Verabreichungsset (300) nach einem der Ansprüche 9 bis 11, wobei das Verabreichungsset (300) ferner eine zweite Fluidleitung (340) umfasst, die mit einem Ende (341) fest mit dem Einlass (201) verbunden ist und mit dem anderen Ende (342) fest mit einem vorgefüllten Flüssigkeitsreservoir (375) verbunden ist, wobei insbesondere das vorgefüllte Reservoir (375) gasfrei mit einer Flüssigkeit vorgefüllt ist.

13. Verabreichungsset (300) nach einem der Ansprüche 9 bis 12, wobei das Verabreichungsset (300) ferner ein Etikett (380) umfasst, das repräsentativ für die Flüssigkeit im vorgefüllten Reservoir (375) ist und zum Auslesen durch ein Etikettenlesegerät (180) einer Medikamentenpumpen-Antriebsvorrichtung (100) geeignet ist.

## Revendications

1. Dispositif de pompe à médicament trochoïdale (200) comprenant :
un corps de pompe (220) ;
un orifice d'entrée (201) pour l'introduction d'un médicament à administrer ;
un orifice de sortie (202) pour l'administration d'un médicament ;
un rotor de pompe (230) ayant une forme triangulaire convexe et une trajectoire excentrique lors de la rotation à l'intérieur du corps de pompe ;
dans lequel le rotor de pompe (230) possède trois sommets équidistants (231), deux quelconques desquels étant reliés par une ligne courbe convexe (232) ;
dans lequel le corps de pompe (220) possède une chambre de pompe (225) ;
dans lequel la chambre de pompe (225) possède un premier lobe (226a) formant une première partie de paroi intérieure (227a) d'un volume de pompe de la chambre de pompe (225) avec une première entrée (221a) en communication fluidique avec l'orifice d'entrée (201) et une première sortie (222a) en communication fluidique avec l'orifice de sortie (202), le rotor de pompe (230) tournant de manière excentrique de sorte que le rotor avec la première partie de paroi forme, lors de la rotation, un premier volume d'aspiration (228a) en communication fluidique avec la première entrée (221a) et un premier volume d'expulsion (229a) en communication fluidique avec la première sortie (222a),
dans lequel le rotor de pompe (230) possède un noyau de rotor rigide (236) et un surmoulage élastomère (237) sur au moins des lignes d'étanchéité le long des sommets et des lignes d'étanchéité le long des lignes courbes convexes,
dans lequel le surmoulage est constitué d'un matériau élastomère d'une première classe de matériaux,
dans lequel la première partie de paroi intérieure (227a) est constituée d'un matériau appartenant à une seconde classe de matériaux,
**caractérisée en ce que**
les dimensions extérieures du rotor de pompe (230) avec le surmoulage (237) sont plus grandes que les dimensions intérieures correspondantes de parties en contact de la chambre de pompe (225),
dans lequel les sommets (231) du rotor de pompe à rotation excentrique (230) tracent une ligne trochoïdale correspondant à une forme trochoïdale équivalente ou supérieure aux dimensions intérieures correspondantes de la chambre de pompe (225)
dans lequel la chambre de pompe (225) a une forme trochoïdale avec ledit premier lobe et un second lobe (226b) formant une seconde partie de paroi intérieure (227b) d'un volume de pompe de la chambre de pompe (225) avec le rotor de pompe (230) tournant de manière excentrique avec les sommets se déplaçant le long d'une ligne trochoïdale correspondant à la forme trochoïdale, formant ainsi, lors de la rotation, un second volume d'aspiration (228b) et un second volume d'expulsion (229b),
dans lequel la seconde partie de paroi intérieure (227b) possède, dans une direction circonférentielle, au moins en section, une encoche (227c) formée dans celle-ci, formant, lors de la rotation du rotor de pompe, une dérivation de compensation de pression entre le second volume d'expulsion (229b) et le second volume d'aspiration (228b).

2. Dispositif de pompe à médicament trochoïdale selon la revendication 1, dans lequel la première classe de matériaux est un groupe constitué par caoutchouc silicone liquide, LSR, caoutchouc éthylène-propylène-diène monomère, EPDM, élastomère thermoplastique, TPE, polyuréthane thermoplastique, PU, caoutchoucs butyle et caoutchoucs nitrile.

3. Dispositif de pompe à médicament trochoïdale selon l'une quelconque des revendications 1 et 2, dans lequel le surmoulage est constitué de caoutchouc de silicone liquide, LSR, d'une dureté comprise entre 10 et 100 Shore (ShA), en particulier entre 50 et 100 Shore (ShA), en particulier entre 60 et 70 Shore (ShA).

4. Dispositif de pompe à médicament trochoïdale selon l'une quelconque des revendications 1 à 3, dans lequel la seconde classe de matériaux est un groupe constitué par polyéthylène téréphtalate, PET, polycarbonate, PC, Tritan, polyéthylène haute densité, HDPE, COC (copolymère de cyclooléfine), PBT, PPS et PEI.

5. Dispositif de pompe à médicament trochoïdale selon l'une quelconque des revendications 1 à 4, dans lequel les dimensions extérieures du rotor de pompe (230) avec le surmoulage sont plus grandes que les dimensions intérieures correspondantes de parties en contact de la chambre de pompe (225) par un facteur, lequel facteur est compris entre 1,01 et 1,1, en particulier entre 1,01 et 1,05, en particulier entre 1,02 et 1,03.

6. Dispositif de pompe à médicament trochoïdale selon la revendication 5, dans lequel le facteur dans la direction radiale est compris entre 1,01 et 1,1, en particulier entre 1,01 et 1,05, en particulier entre 1,02 et 1,03.

7. Dispositif de pompe à médicament trochoïdale selon l'une quelconque des revendications 5 et 6, dans lequel le facteur dans la direction axiale est compris entre 1,01 et 1,1, en particulier entre 1,01 et 1,05, en particulier entre 1,01 et 1,03.

8. Dispositif de pompe à médicament trochoïdale selon l'une quelconque des revendications 1 à 7, dans lequel la chambre de pompe (225) dans le volume de pompe du second lobe (226b) possède une seconde entrée (221b) en communication fluidique avec l'orifice d'entrée (201) et une seconde sortie (222b) en communication fluidique avec l'orifice de sortie (202) avec le rotor de pompe (230) tournant de manière excentrique de sorte qu'au moins trois parties distinctes (231, 232) du rotor (230) soient en contact étanche permanent avec la première partie de paroi intérieure (227a) du premier lobe (226a) et la seconde partie de paroi intérieure (227b) du second lobe (226b), formant ainsi, lors de la rotation, un second volume d'aspiration (228b) en communication fluidique avec la seconde entrée (221b) et un second volume d'expulsion (229b) en communication fluidique avec la seconde sortie (222b), dans lequel, en particulier, la seconde entrée (221b) est en communication fluidique avec l'orifice d'entrée (201) et la seconde sortie (222b) est en communication fluidique avec l'orifice de sortie (202).

9. Kit de perfusion (300) destiné à fournir un médicament à partir d'un réservoir à un patient (400), le kit de perfusion (300) comprend :
une pompe à médicament trochoïdale (200) selon l'une quelconque des revendications 1 à 8 ;
un premier conduit de fluide (310) dont une extrémité (312) est reliée de manière fixe à l'orifice de sortie (202) et dont l'autre extrémité (311) est reliée de manière fixe à embout Luer-Lok (350).

10. Kit de perfusion selon la revendication 9, dans lequel le premier conduit de fluide (310) a une section amont (320) et une section aval flexible (330), dans lequel la section aval flexible (330) est plus flexible que la section amont (320), dans lequel en particulier le premier conduit de fluide (310) est fait d'un tube flexible de diamètre et d'épaisseur de paroi sensiblement constants, et la section aval flexible (330) est formée par un enroulement hélicoïdal préformé (335) du tube flexible, la géométrie de celui-ci fournissant une flexibilité plus élevée qu'une partie non hélicoïdale.

11. Kit de perfusion selon l'une quelconque des revendications 9 à 10, dans lequel le kit de perfusion (300) comprend en outre un second conduit de fluide (340) dont une extrémité (341) est reliée de manière fixe à l'orifice d'entrée (201) et dont l'autre extrémité (342) est reliée de manière fixe à un raccord (360) conçu pour être relié de manière amovible à un réservoir de fluide (370).

12. Kit de perfusion selon l'une quelconque des revendications 9 à 11, dans lequel le kit de perfusion (300) comprend en outre un second conduit de fluide (340) dont une extrémité (341) est reliée de manière fixe à l'orifice d'entrée (201) et dont l'autre extrémité (342) est reliée de manière fixe à un réservoir de fluide prérempli (375), dans lequel en particulier le réservoir prérempli (375) est prérempli, sans volume de gaz, avec un liquide..

13. Kit de perfusion selon l'une quelconque des revendications 9 à 12, dans lequel le kit de perfusion (300) comprend en outre une étiquette (380) spécifiant le liquide dans le réservoir prérempli (375) et adaptée pour être lue par un lecteur d'étiquettes (180) d'un dispositif d'entraînement de pompe à médicament (100).
